# FASCICULE DE BREVET EUROPEEN

(11) **EP 2 078 512 B1**
(45) Date de publication et mention de la délivrance du brevet: **18.07.2012**
(21) Numéro de dépôt: 08291244.5
(22) Date de dépôt: 29.12.2008
(51) Int. Cl.: A61F 2/82, D03D 3/02, A61F 2/86

(54) **Système permettant d'obturer un anévrisme ou analogue dans un vaisseau sanguin**
System zum Verschließen eines Aneurysmas oder ähnlichen Gebildes in einem Blutgefäß
System for blocking an aneurysm or similar in a blood vessel

(30) Priorité: 14.01.2008 FR 0800174
(43) Date de publication de la demande: 15.07.2009
(73) Titulaire: Balt Extrusion, 95160 Montomorency (FR)
(72) Inventeur: Plowiecki, Nicolas, 95160 Montmorency (FR)
(74) Mandataire: Flavenot, Bernard

(56) Documents cités:
- EP-A- 0 804 909
- EP-A- 1 532 943
- WO-A-2004/110304
- US-A- 5 545 208
- US-A1- 2003 149 475
- US-A1- 2005 038 503

## Description

La présente invention concerne les systèmes permettant d'obturer un anévrisme ou analogue dans un vaisseau sanguin comme une artère, qui sont connus des techniciens sous le terme de "stent", et qui trouvent une application particulièrement avantageuse, mais non exclusivement, pour l'obturation d'anévrismes dans des vaisseaux de très petite section, comme ceux que l'on trouve dans le cerveau.

Un anévrisme est une dilatation d'un vaisseau sanguin ou analogue. Pour soigner un anévrisme on utilise couramment un système désigné par les techniciens sous le terme "stent" qui, pour simplifier la description suivante et en faciliter la compréhension, sera parfois utilisé pour désigner un système permettant d'obturer un anévrisme ou analogue dans un vaisseau sanguin comme une artère ou analogue.

Un stent comporte essentiellement un manchon destiné à épouser la paroi intérieure du vaisseau en débordant de part et d'autre de l'anévrisme de façon à l'obturer complètement.

Pour implanter un tel manchon dans un vaisseau sanguin, la solution la plus adaptée est de l'introduire en un endroit où le vaisseau est le plus accessible depuis l'extérieur du corps humain, puis de le faire progresser dans le vaisseau jusqu'à l'endroit de l'anévrisme.

Pour arriver à une telle implantation selon cette procédure, la structure de stent idéale serait celle d'un manchon ayant une paroi pleine et apte à prendre deux formes, une forme repliée dans laquelle le manchon a une dimension transversale d'une première valeur donnée inférieure à celle du vaisseau qu'il doit parcourir, et une forme dépliée mémorisée, dans laquelle le manchon a une dimension transversale d'une seconde valeur donnée au moins égale à celle du vaisseau.

Or, une telle structure de manchon est actuellement impossible à réaliser. Pour pallier cet inconvénient, on réalise un manchon constitué d'une pluralité de fils à mémoire de forme tissés en formant des spires de forme sensiblement hélicoïdale se chevauchant alternativement les unes les autres. Le système peut en outre comporter un support central sur lequel est emmanché le manchon.

Avec une telle structure de manchon, le manchon sur son support est introduit dans le vaisseau sous sa forme repliée, c'est-à-dire sa forme dans laquelle les spires en hélice sont très allongées, en étant maintenu dans une gaine comme un cathéter ou analogue. Lorsque le stent est arrivé à l'endroit de l'anévrisme, la gaine est retirée et, du fait que les fils constituant le manchon sont à mémoire de forme, le manchon se déplie automatiquement et vient se plaquer contre la paroi intérieure du vaisseau sanguin devant l'entrée de l'anévrisme. Il se forme alors des espaces libres entre les fils, sensiblement en forme de losanges, mais, avec le temps, ces espaces libres s'obturent notamment par les fibrines et autres produits coagulants du sang, et l'anévrisme est donc lui-même obturé, ce qui est le but de ces systèmes.

Il est connu un tel système comme celui qui est décrit dans le US 2005/038503 A1, qui comporte notamment un manchon cylindrique ayant une section transversale comprise entre 3 et 6 mm et constitué d'une pluralité de fils à mémoire de forme tissés en nombre de l'ordre de cinquante.

Cette structure donne satisfaction aux praticiens, mais il est bien évident que toute amélioration des systèmes décrits ci-dessus ne peut qu'être appréciée, ne serait-ce que pour le confort et la sécurité des patients, ainsi que la fiabilité du traitement.

Aussi, la présente invention a-t-elle pour but de réaliser un perfectionnement au système du type décrit ci-dessus permettant d'obturer un anévrisme ou analogue dans un vaisseau sanguin comme une artère, pour obtenir un système qui permet d'obturer encore plus rapidement un anévrisme ou analogue, et dont la mise en place est encore plus aisée et facilement contrôlée qu'avec les systèmes similaires de l'antérieur.

Plus précisément, la présente invention a pour objet un système permettant d'obturer un anévrisme ou analogue dans un vaisseau sanguin comme une artère, selon la revendication 1 annexée à la présente description.

D'autres caractéristiques et avantages de l'invention apparaîtront au cours de la description suivante donnée en regard des dessins annexés à titre illustratif mais nullement limitatif, dans lesquels :
La figure 1 représente une vue de côté d'une partie du système perfectionné selon l'invention permettant d'obturer un anévrisme ou analogue dans un vaisseau sanguin comme une artère, selon un mode de réalisation, et
La figure 2 représente une vue en coupe longitudinale du système perfectionné selon l'invention, selon un autre mode de réalisation comportant la réalisation selon la figure 1.

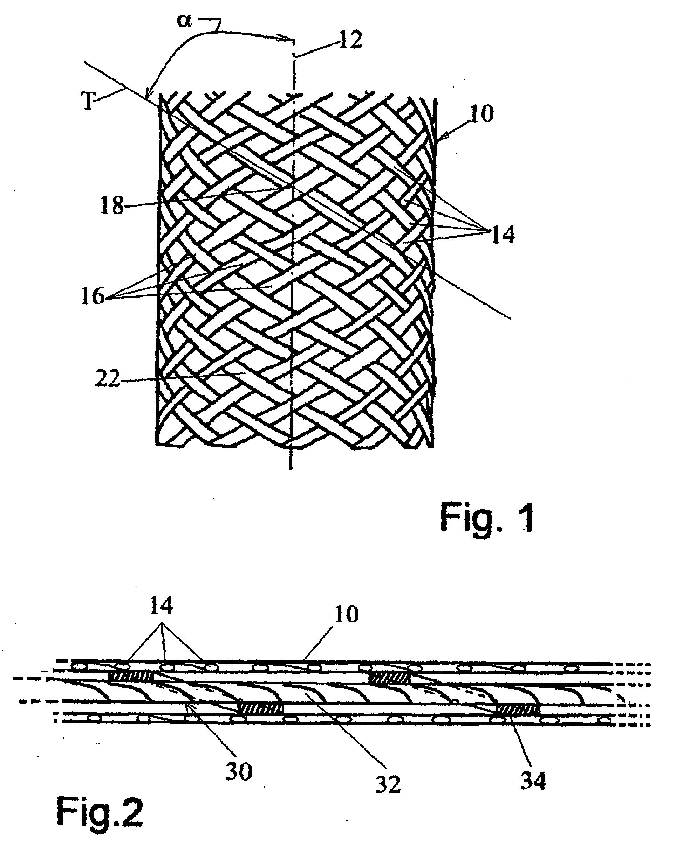

Plus particulièrement en référence à la figure 1, le système selon l'invention permettant d'obturer un anévrisme ou analogue dans un vaisseau sanguin comme une artère, ou "stent", comporte au moins un manchon cylindrique 10 défini selon un axe longitudinal 12.

Ce manchon est agencé de façon à être apte à prendre deux formes, une forme repliée dans laquelle il a une dimension transversale d'une première valeur donnée de l'ordre de 0,6 mm et une forme dépliée dans laquelle il a une dimension transversale d'une seconde valeur donnée comprise entre 2 mm et 6 mm. Le manchon est constitué d'une pluralité de fils 14 à mémoire de forme, tissés en formant des spires 16 de forme sensiblement hélicoïdale se chevauchant alternativement les unes les autres.

Il est précisé que, par "fil à mémoire de forme", on entend un fil qui, après avoir été déformé, reprend automatiquement sa forme initiale lorsque cesse l'action qui a provoqué sa déformation, changement de la température, déformation élastique, etc. Mais de façon avantageuse ces fils à mémoire de forme 14 seront réalisés dans un alliage de Nickel et de Titane connu sous la Marque de commerce Nitinol^{®},

Selon une caractéristique importante de l'invention, le nombre de fils 14 à mémoire de forme est supérieur à quarante, la section transversale de chaque fil en alliage à mémoire est inférieure à 45 µm et, lorsque le manchon est dans sa forme dépliée, l'angle α que forme la tangente T en tout point 18 d'une spire par rapport au plan contenant l'axe longitudinal 12 du manchon 10 et le point 18 de la spire sur lequel est définie la tangente, est supérieur à 60 degrés.

Avec la structure de stent telle que définie ci-dessus, la Demanderesse a obtenu de très bons résultats. Le manchon du système selon l'invention présente en quelque sorte des caractéristiques fonctionnelles qui peuvent être comparables à celles d'un manchon qui peut prendre deux formes, à savoir repliée et dépliée, et celles du manchon idéal qui aurait une paroi étanche, comme évoqué dans le préambule de la présente description.

Les fils peuvent être de toute forme, plats, en bande, cylindriques de révolution, etc. Par "dimension transversale", on entend, dans le cas d'un fil cylindrique de révolution, le diamètre de ce fil, dans le cas d'un fil plat ou en bande, la largeur du fil la plus grande, et, pour toute autre forme possible, le diamètre du cylindre de révolution enveloppant virtuellement cette forme.

Selon une réalisation avantageuse, pour obtenir un manchon présentant à la fois une certaine souplesse et une certaine rigidité pour faciliter sa progression dans le vaisseau, son dépliement et son plaquage contre la paroi intérieure du vaisseau quelle que soit la forme de ce vaisseau, même courbée, la pluralité de fils 14 à mémoire de forme comprend au moins deux premier et second ensembles de fils, les fils du premier ensemble ayant une section d'une valeur différente de celle des fils du second ensemble, en respectant la caractéristique définie ci-dessus, à savoir que les deux valeurs de section doivent être inférieures à 45 µm. L'homme du métier saura, notamment expérimentalement, déterminer la valeur de ces deux sections, par exemple 40 µm et 30 µm.

Selon une réalisation qui a donné toute satisfaction à la Demanderesse, les premier et second ensembles de fils 14 à mémoire de forme ont le même nombre de fils et sont tissés de façon alternée.

En outre, pour pouvoir contrôler, par radiographie, la mise en place du manchon 10, il est prévu au moins un fil 22 en matériau radio-opaque, ce fil 22 en matériau radio-opaque étant tissé avec les fils 14 à mémoire de forme en formant des spires 16 sensiblement hélicoïdales.

Cependant, de façon avantageuse, pour que ce fil 22 en matériau radio-opaque qui est en général plus rigide que les fils 14 à mémoire de forme définis ci-dessus, ne tende pas à trop rigidifier le manchon, ce fil 22 en matériau radio-opaque est constitué d'une pluralité de brins torsadés les uns avec les autres, par exemple au nombre de quatre ou cinq.

De façon préférentielle, il est cependant prévu que ce manchon comporte une pluralité de fils 22 en matériau radio-opaque répartis de façon relativement régulière dans la pluralité de fils 14 à mémoire de forme, par exemple au moins trois fils 22 en matériau radio-opaque, et qu'au moins un fil 22, et avantageusement les trois, soit en un matériau comme du Platine.

En outre, par sécurité, pour que le manchon vienne se plaquer contre la paroi du vaisseau au niveau de l'anévrisme quelle que soit la forme du vaisseau, même en courbe, le manchon est conformé de façon que, lorsqu'il est dans sa forme dépliée, au moins l'une de ses extrémités, avantageusement les deux, s'ouvre selon une forme évasée, par exemple en forme de trompette ou analogue.

Par référence maintenant à la figure 2 prise en combinaison avec la figure 1, pour faciliter son introduction dans une gaine comme un cathéter ou analogue et sa translation dans cette gaine jusqu'à l'endroit de l'anévrisme à obturer, le système comporte en outre un support central 30 enfiché dans le manchon 10, ce support central 30 comprenant une âme souple 32 et une bande 34 enroulée à spires non jointives sur l'âme souple 32, la bande étant réalisée en un matériau à coefficient de friction relativement élevé et étant au contact de la paroi intérieure du manchon lorsque ce dernier est dans sa position repliée.

Selon une réalisation préférentielle, le matériau à coefficient de friction relativement élevé est choisi parmi l'un des matériaux suivants : latex, mélange de latex et d'un autre matériau, caoutchouc, matière plastique élastique.

## Revendications

1. Système permettant d'obturer un anévrisme ou analogue dans un vaisseau sanguin comme une artère, comportant au moins un manchon cylindrique (10) défini selon une axe longitudinal (12), ledit manchon étant agencé de façon à être apte à prendre deux formes, une forme repliée dans laquelle ledit manchon a une dimension transversale d'une première valeur donnée de l'ordre de 0,6 mm et une forme dépliée dans laquelle ledit manchon a une dimension transversale d'une seconde valeur donnée comprise entre 2 mm et 6 mm, ledit manchon étant constitué d'une pluralité de fils (14) à mémoire de forme tissés en formant des spires (16) de forme sensiblement hélicoïdale se chevauchant alternativement les unes les autres, le nombre de fils (14) à mémoire de forme étant supérieur à quarante et la section transversale de chaque fil à mémoire de forme étant inférieure à 45 µm, **caractérisé par le fait que**, lorsque le manchon est dans sa forme dépliée, l'angle (α) que forme la tangente (T) en tout point (18) d'une spire par rapport au plan contenant l'axe longitudinal (12) du manchon (10) et le point (18) de ladite spire sur lequel est définie ladite tangente, est supérieur à 60 degrés.

2. Système selon la revendication 1, **caractérisé par le fait que** ladite pluralité de fils (14) à mémoire de forme comprend au moins deux premier et second ensembles de dits fils, les fils du premier ensemble ayant une section d'une valeur différente de celle des fils du second ensemble.

3. Système selon l'une des revendications 1 et 2, **caractérisé par le fait que** lesdits fils (14) à mémoire de forme sont réalisés dans un alliage de Nickel et de Titane (Nitinol^{®}).

4. Système selon l'une des revendications 1 à 3, **caractérisé par le fait qu'**il comporte en outre au moins un fil (22) en matériau radio-opaque, ledit fil en matériau radio-opaque (22) étant tissé avec les fils (14) à mémoire de forme en formant des spires (16) sensiblement hélicoïdales.

5. Système selon la revendication 4, **caractérisé par le fait que** ledit fil (22) en matériau radio-opaque est constitué d'une pluralité de brins torsadés les uns avec les autres.

6. Système selon l'une des revendications 4 et 5, **caractérisé par le fait qu'**il comporte une pluralité de fils (22) en matériau radio-opaque répartis de façon relativement régulière dans ladite pluralité de fils (14) à mémoire de forme.

7. Système selon la revendication 6, **caractérisé par le fait qu'**il comporte au moins trois fils (22) en matériau radio-opaque.

8. Système selon l'une des revendications 4 à 7, **caractérisé par le fait qu'**au moins un fil (22) en matériau radio-opaque est en Platine.

9. Système selon l'une des revendications précédentes, **caractérisé par le fait que** ledit manchon est conformé de façon que, lorsqu'il est dans sa forme dépliée, au moins l'une de ses extrémités s'ouvre selon une forme évasée.

10. Système selon l'une des revendications 1 à 9, **caractérisé par le fait qu'**il comporte en outre un support central (30) enfiché dans ledit manchon (10), ledit support central (30) comprenant une âme souple (32) et une bande (34) enroulée à spires non jointives sur ladite âme souple (32), ladite bande étant réalisée en un matériau à coefficient de friction relativement élevé et étant au contact de la paroi intérieure du dit manchon lorsque ce dernier est dans sa position repliée.

11. Système selon la revendication 10, **caractérisé par le fait que** ledit matériau à coefficient de friction relativement élevé est l'un des matériaux suivants : latex, mélange de latex et d'un autre matériau, caoutchouc, matière plastique élastique.

12. Système selon l'une des revendications 2 à 9 quand elle dépend de la revendication 2, **caractérisé par le fait que** les premier et second ensembles de fils (14) à mémoire de forme ont le même nombre de fils.

## Claims

1. A system enabling an aneurysm or the like to be closed in a blood vessel such as an artery, the system comprising at least one cylindrical sleeve (10) defined along a longitudinal axis (12), said sleeve being arranged in such a manner as to be suitable for taking on two shapes, a folded shape in which said sleeve presents a transverse dimension with a first given value of the order of 0.6 mm, and an unfolded shape in which said sleeve has a transverse dimension of a second given value lying in the range 2 mm to 6 mm, said sleeve being made up of a plurality of shape-memory wires (14) forming turns (16) of substantially helical shape that overlap one another in alternation, the number of shape-memory wires (14) being greater than forty and the cross-section of each shape-memory wire being less than 45 µm, the system being **characterized by** the fact that, when the sleeve is in its unfolded shape, the angle (α) between the tangent (T) at any point (18) on a turn relative to the plane containing the longitudinal axis (12) of the sleeve (10) and the point (18) of said turn at which said tangent is defined is greater than 60 degrees.

2. A system according to claim 1, **characterized by** the fact that said plurality of shape-memory wires (14) comprises at least first and second sets of said wires, the wires of the first set having a section of a value that is different from the section of the wires of the second set.

3. A system according to claim 1 or claim 2, **characterized by** the fact that said shape-memory wires (14) are made of an alloy of nickel and titanium (Nitinol®).

4. A system according to any one of claims 1 to 3, **characterized by** the fact that it further includes at least one wire (22) of a radio-opaque material, said wire (22) of radio-opaque material being woven with the shape-memory wires (14) forming substantially helical turns (16).

5. A system according to claim 4, **characterized by** the fact that said wire (22) of radio-opaque material is constituted by a plurality of strands that are mutually twisted together.

6. A system according to claim 4 or claim 5, **characterized by** the fact that it includes a plurality of wires (22) of radio-opaque material that are distributed in relatively regular manner amongst said plurality of shape-memory wires (14).

7. A system according to claim 6, **characterized by** the fact that it includes at least three wires (22) of radio-opaque material.

8. A system according to any one of claims 4 to 7, **characterized by** the fact that at least one wire (22) of radio-opaque material is made of platinum.

9. A system according to any preceding claim, **characterized by** the fact that said sleeve is shaped in such a manner that when it is in its unfolded shape at least one of its ends opens with a flared shape.

10. A system according to any one of claims 1 to 9, **characterized by** the fact that it further includes a central support (30) engaged in said sleeve (10), said central support (30) comprising a flexible core (32) and a strip (34) wound with non-touching turns on said flexible core (32), said strip being made of a material having a relatively high coefficient of friction and being in contact with the inside wall of said sleeve when the sleeve is in its folded position.

11. A system according to claim 10, **characterized by** the fact that said material having a relatively high coefficient of friction is one of the following materials: latex; a mixture of latex and some other material; rubber; and elastic plastics material.

12. A system according to any one of claims 2 to 9 when dependent on claim 2, **characterized by** the fact that the first and second sets of shape-memory wire (14) both have the same number of wires.

## Patentansprüche

1. System, das ermöglicht, ein Aneurysma oder dergleichen in einem Blutgefäß wie etwa einer Arterie zu verschließen, das wenigstens eine zylindrische Muffe (10) umfasst, die längs einer Längsachse (12) definiert ist, wobei die Muffe so beschaffen ist, dass sie zwei Formen annehmen kann, nämlich eine zusammengefaltete Form, in der die Muffe eine transversale Abmessung mit einem ersten gegebenen Wert in der Größenordnung von 0,6 mm hat, und eine auseinandergefaltete Form, in der die Muffe eine transversale Abmessung mit einem zweiten gegebenen Wert im Bereich von 2 mm bis 6 mm hat, wobei die Muffe aus mehreren gewebten Drähten (14) mit Formerinnerungsvermögen gebildet ist, indem im Wesentlichen schraubenlinienförmige Windungen (16) gebildet sind, die sich abwechselnd überlappen, wobei die Anzahl der Drähte (14) mit Formerinnerungsvermögen größer als vierzig ist und der transversale Querschnitt jedes Drahts mit Formerinnerungsvermögen kleiner als 45 µm ist, **dadurch gekennzeichnet, dass** dann, wenn die Muffe ihre auseinandergefaltete Form hat, der Winkel (α), den die Tangente (T) an jedem Punkt (18) einer Windung in Bezug auf die die Längsachse (12) der Muffe (10) und den Punkt (18) der Windung, an dem die Tangente definiert ist, bildet, größer als 60 Grad ist.

2. System nach Anspruch 1, **dadurch gekennzeichnet, dass** die mehreren Drähte (14) mit Formerinnerungsvermögen wenigstens eine erste und eine zweite Gesamtheit von Drähten umfassen, wobei die Drähte der ersten Gesamtheit einen Querschnitt mit einem Wert haben, der von jenem der Drähte der zweiten Gesamtheit verschieden ist.

3. System nach einem der Ansprüche 1 und 2, **dadurch gekennzeichnet, dass** die Drähte (14) mit Formerinnerungsvermögen aus einer Legierung aus Nickel und Titan (Nitinol^{®}) hergestellt sind.

4. System nach einem der Ansprüche 1 bis 3, **dadurch gekennzeichnet, dass** es außerdem wenigstens einen Draht (22) aus einem strahlenundurchlässigen Material umfasst, wobei der Draht aus einem strahlenundurchlässigen Material (22) mit den Drähten (14) mit Formerinnerungsvermögen verwebt ist, indem im Wesentlichen schraubenlinienförmige Windungen (16) gebildet sind.

5. System nach Anspruch 4, **dadurch gekennzeichnet, dass** der Draht (22) aus einem strahlenundurchlässigen Material aus mehreren Fasern, die miteinander verdrillt sind, gebildet ist.

6. System nach einem der Ansprüche 4 und 5, **dadurch gekennzeichnet, dass** es mehrere Drähte (22) aus einem strahlenundurchlässigen Material umfasst, die verhältnismäßig regelmäßig unter den mehreren Drähten (14) mit Formerinnerungsvermögen verteilt sind.

7. System nach Anspruch 6, **dadurch gekennzeichnet, dass** es wenigstens drei Drähte (22) aus strahlenundurchlässigem Material umfasst.

8. System nach einem der Ansprüche 4 bis 7, **dadurch gekennzeichnet, dass** wenigstens ein Draht (22) aus strahlenundurchlässigem Material aus Platin ist.

9. System nach einem der vorhergehenden Ansprüche, **dadurch gekennzeichnet, dass** die Muffe so beschaffen ist, dass dann, wenn sie in ihrer auseinandergefalteten Form ist, wenigstens eines ihrer Enden sich gemäß einer sich erweiternden Form öffnet.

10. System nach einem der Ansprüche 1 bis 9, **dadurch gekennzeichnet, dass** es außerdem einen Mittelträger (30) umfasst, der in die Muffe (10) gesteckt ist, wobei der Mittelträger (30) einen nachgiebigen Kern (32) und ein gewickeltes Band (34) mit nicht verbundenen Windungen auf dem nachgiebigen Kern (32) umfasst, wobei das Band aus einem Material mit verhältnismäßig hohem Reibkoeffizienten hergestellt ist und mit der Innenwand der Muffe in Kontakt ist, wenn diese letztere in ihrer zusammengefalteten Position ist.

11. System nach Anspruch 10, **dadurch gekennzeichnet, dass** das Material mit verhältnismäßig hohem Reibkoeffizienten eines der folgenden Materialien ist: Latex, Gemisch aus Latex und einem anderen Material, Kautschuk, elastischer Kunststoff.

12. System nach einem der Ansprüche 2 bis 9, wenn abhängig von Anspruch 2, **dadurch gekennzeichnet, dass** die erste und die zweite Gesamtheit von Drähten (14) mit Formerinnerungsvermögen die gleiche Anzahl von Drähten besitzen.
